# EUROPÄISCHE PATENTSCHRIFT

(11) **EP 4 114 500 B1**
(45) Veröffentlichungstag und Bekanntmachung des Hinweises auf die Patenterteilung: **24.12.2025**
(21) Anmeldenummer: 21710219.3
(22) Anmeldetag: 03.03.2021
(51) Int. Cl.: A61M 39/16, A61M 39/10, A61M 39/26

(54) **KUPPLUNGSELEMENT FÜR EIN GESCHLOSSENES FLUIDTRANSFERSYSTEM, GEGENKUPPLUNGSELEMENT FÜR EIN SOLCHES KUPPLUNGSELEMENT SOWIE KUPPLUNGSSYSTEM**
COUPLING ELEMENT FOR A CLOSED FLUID TRANSFER SYSTEM, COUNTER COUPLING ELEMENT FOR A COUPLING ELEMENT OF THIS TYPE, AND COUPLING SYSTEM
ÉLÉMENT D'ACCOUPLEMENT POUR UN SYSTÈME DE TRANSFERT FLUIDIQUE FERMÉ, ÉLÉMENT D'ACCOUPLEMENT HOMOLOGUE POUR UN TEL ÉLÉMENT D'ACCOUPLEMENT ET SYSTÈME D'ACCOUPLEMENT

(30) Priorität: 06.03.2020 DE 102020202935
(43) Veröffentlichungstag der Anmeldung: 11.01.2023
(73) Patentinhaber: B. Braun Melsungen AG, 34212 Melsungen (DE)
(72) Erfinder: KOPP, Florin, 26419 Schortens (DE); BERG, Karl Martin, 34212 Melsungen (DE); KRUG-SAUER, Konstantin, 34281 Gudensberg (DE); BOLZ, Johannes, 34132 Kassel (DE)
(74) Vertreter: Maiwald GmbH
(86) Internationale Anmeldenummer: PCT/EP2021/055249
(87) Internationale Veröffentlichungsnummer: WO 2021/175884

(56) Entgegenhaltungen:
- US-A1- 2011 106 046
- US-A1- 2015 126 958
- US-A1- 2019 184 152

## Beschreibung

Die Erfindung betrifft ein Kupplungselement für ein geschlossenes Fluidtransfersystem, ein Gegenkupplungselement für ein solches Kupplungselement sowie ein Kupplungssystem.

Viele Substanzen, die als Injektionen oder in vergleichbarer Zuführungsform verabreicht werden, wie CMR-Arzneimittel, die beispielsweise in der Krebstherapie eingesetzt werden und bei ihrer therapeutischen Anwendung vor allem auf die Schädigung wachstumsintensiver Tumorzellen gerichtet sind, weisen außerhalb der eigentlichen Therapieanwendung ein erhebliches Gefährdungspotential auf. So sind solche Substanzen bedingt durch ihren Wirkmechanismus zum Teil selbst krebserzeugend, weshalb der Kontakt mit nicht in Therapie befindlichen Personen zu vermeiden ist. Für CMR-Arzneimittel werden daher bei der Herstellung applikationsfertiger Zubereitungen vermehrt geschlossene Medikamententransfersystem, sogenannte "Closed System Transfer Devices" oder auch CSTDs eingesetzt. Wichtiger Bestandteil solcher CSTDs sind Kupplungssysteme, die den sicheren Transfer von CMR-Arzneimitteln oder anderen Substanzen ermöglichen und nach dem Trennen der Verbindung trocken abschließen, um so die Umgebung vor einer Kontamination, z.B. durch Leckagen oder Tropfenbildung auf den Oberflächen der Kupplungselemente, zu schützen.

Kupplungssystem dieser Art werden im Allgemeinen mit den Begriffen "dry connection", "automatic self-sealing technology" oder "closed connection" in Verbindung gebracht und essentiell für die Realisierung geschlossener Fluidtransfersysteme.

Bekannte Kupplungssysteme sind in ihrer Handhabung und Verbindungsstruktur häufig komplex und können schlechte Durchflussraten aufweisen. Zudem können Fluidrückstände an den Dichtflächen bzw. Kupplungsflächen auftreten, wenn keine konstante Flächenpressung der Dichtflächen aufrechterhalten wird.

Allen derzeitigen Systemen ist darüber hinaus gemein, dass sie vergleichsweise groß sind, was zu Nachteilen bei einer Anwendung in Patientennähe führt, und zumindest ein Kupplungselement oder eine Gegenkupplungselement strukturbedingt eine schlechte Zugänglichkeit der Kupplungs- bzw. Gegenkupplungsfläche aufweist, so dass eine Desinfektion erschwert wird.

Die US 2019/184152 A1 betrifft ein männliches Verbindungsstück, das mit einem weiblichen Verbindungsstück verbindbar ist. Das männliche Verbindungsstück umfasst ein rohrförmiges Strömungspfadelement, das eine Öffnung an einem Endabschnitt aufweist, einen Ventilkörper, der die Öffnung schließt, einen Gehäusehauptkörper, und einen Bewegungskörper, der sich in Bezug auf den Gehäusehauptkörper bewegt und den Ventilkörper verformt, um einen Modus zwischen einem ersten Modus, in dem die Öffnung durch den Ventilkörper geschlossen ist, und einem zweiten Modus, in dem die Öffnung von dem Ventilkörper geöffnet ist, zu ändern.

Die US 2011/106046 A1 betrifft eine Verbinderanordnung mit einem ersten Verbinder, der mit einem ersten Verbinderkörper, einer Hohlnadel, die an der Innenseite des ersten Verbinderkörpers gelagert ist und ein Seitenloch aufweist, und einem ersten Dichtungselement versehen ist, das einen Kopf aufweist, der von der Hohlnadel durchstochen werden kann. Die Verbinderanordnung umfasst zudem einem zweiten Verbinder, der mit einem zweiten Verbinderkörper und auch mit einem zweiten Dichtungselement versehen ist, das, wenn es in einem montierten Zustand ist, von der Hohlnadel durchstochen werden kann.

Die US 2015/126958 A1 betrifft ein medizinisches Verbindersystem mit einem ersten Verbinder und einem zweiten Verbinder. Der erste Verbinder weist ein Gehäuse, ein Vorspannelement und mindestens einen Vorsprung auf. Der zweite Verbinder weist mindestens eine Nut zur Aufnahme des mindestens einen Vorsprungs auf. Das proximale Ende des zweiten Verbinders ist konfiguriert, um zumindest teilweise innerhalb des distalen Endes des Gehäuses des ersten Verbinders angeordnet zu sein.

In Anbetracht der mit dem Stand der Technik verbundenen Nachteile ist es Aufgabe der vorliegenden Erfindung, ein Kupplungselement, ein Gegenkupplungselement sowie ein Kupplungssystem für ein geschlossenes Fluidtransfersystem bereitzustellen, bei dem die jeweiligen Kupplungsflächen im dekonnektierten Zustand trocken abschließen und in einfacher Weise sicher handhabbar sind.

Die erfindungsgemäße Aufgabe wird durch ein Kupplungselement für ein geschlossenes Fluidtransfersystem nach Anspruch 1 sowie ein Kupplungssystem nach Anspruch 12 gelöst. Weitere vorteilhafte Ausgestaltungen der Erfindung ergeben sich aus den Unteransprüchen.

Erfindungsgemäß umfasst das Kupplungselement für ein geschlossenes Fluidtransfersystem ein Kupplungselementgehäuse mit einem Fluidanschluss und einer Kupplungsseite, wobei das Kupplungselementgehäuse eine sich von dem Fluidanschluss in Richtung der Kupplungsseite erstreckende Längsachse aufweist, einen Dorn mit zumindest einer Fluidöffnung, der in einer am Fluidanschluss angeordneten Dornaufnahme des Gehäuses gehalten wird und sich in Richtung der Längsachse in das Kupplungselementgehäuse hinein erstreckt, wobei die zumindest eine Fluidöffnung in einem der Kupplungsseite zugewandten Endabschnitt des Dorns angeordnet ist, eine in dem Kupplungselementgehäuse auf der Kupplungsseite angeordnete Dichtelementaufnahme und ein Dichtelement, das in der Dichtelementaufnahme angeordnet ist, wobei das Kupplungsgehäuse einen Gehäuseabschnitt aufweist, der die Dichtelementaufnahme in Bezug auf die Längsachse in axialer Richtung zumindest abschnittsweise umgibt und auf der dem Dichtelement zugewandten Innenseite einen Kupplungsgehäusegewindeabschnitt aufweist, und wobei die Dichtelementaufnahme mit dem Dichtelement in Richtung der Längsachse über den Kupplungsgehäusegewindeabschnitt zwischen einer Position mit maximalem Abstand zum Fluidanschluss und einer Position mit minimalem Abstand zum Fluidanschluss über eine Dichtelementaufnahmeführungsstruktur geführt bewegbar ist.

Durch das Zusammenwirken der Dichtelementaufnahmeführungsstruktur mit dem Kupplungsgehäusegewindeabschnitt kann die Dichtelementaufnahme gegen eine unbeabsichtigte Bewegung durch reine Druck- oder Zugkräfte gesichert werden. Die Position mit maximalem Abstand zum Fluidanschluss korrespondiert zu einer Position der Dichtelementaufnahme in einem dekonnektierten Zustand, während die Position mit minimalem Abstand zum Fluidanschluss bevorzugt dann vorliegt, wenn ein konnektierter Zustand erreicht wird, der für eine fluide Verbindung mit einem Gegenkupplungselement vorgesehen ist.

Der Begriff "Gewinde" in Bezug auf den Kupplungsgehäusegewindeabschnitt oder auch im Folgenden ist nicht auf ein isometrisches Gewinde oder ein Gewinde mit schrägen Gewindeflanken beschränkt, sondern umfasst allgemein eine Strukturausbildung, bei der eine Führungsstruktur wendelartig ausgebildet wird.

Zudem weist die Dichtelementaufnahme an ihrem der Kupplungsseite zugewandten Ende zumindest zwei in Richtung der Längsachse in Richtung der Kupplungsseite vorstehende Schnapphaken als eine kupplungselementseitige Befestigungsstruktur für ein Gegenkupplungselement auf.

Durch die kupplungselementseitige Befestigungsstruktur an der Dichtelementaufnahme kann sichergestellt werden, dass ein hieran befestigtes Gegenkupplungselement über die Bewegung der Dichtelementaufnahme von einer Position mit maximalem Abstand zum Fluidanschluss und minimalen Abstand zum Fluidanschluss entlang der Längsachse in einer vorbestimmten Positionsbeziehung gehalten wird. Insbesondere weist eine solche vorbestimmte Positionsbeziehung eine konstante Flächenpressung zwischen dem Dichtelement und dem Gegenkupplungselementdichtelement auf, so dass eine Fluiddichtheit sichergestellt werden kann. Mit anderen Worten wird über den gesamten Konnektierungsweg eine konstante Flächenpressung der Elastomerflächen erzielt. Bevorzugt ist die Befestigungsstruktur so ausgelegt, dass die Befestigung des Gegenkupplungselements an der Befestigungsstruktur lediglich über die Aufbringung von Druckkräften in Richtung des Kupplungselements und/oder Gegenkupplungselement erfolgt und keine Drehbewegung ausgeführt wird, um die Dichtelementaufnahme nicht bereits während des Befestigungsvorgangs entlang des Gewindegangs zu bewegen. Sofern der Befestigungsvorgang eine Drehbewegung umfasst, sollte diese zumindest der Drehbewegung zur Bewegung der Dichtelementaufnahme entlang des Gewindegangs entgegengesetzt sein.

Die Verwendung von Schnapphaken als Befestigungsstruktur setzt beispielsweise eine Befestigung über eine Druckkraft in einfacher Weise um. Zudem stören Schnapphaken die Zugänglichkeit zur kupplungsseitigen Stirnfläche des Kupplungselements kaum, so dass eine Desinfektion weiterhin leicht möglich ist. Darüber hinaus kann ein Verkippen des Gegenkupplungselements durch zumindest zwei Schnapphaken, die bevorzugt einander gegenüberliegend angeordnet sind, verhindert werden.

Insbesondere ist die zumindest eine Fluidöffnung im Dichtelement angeordnet ist, wenn sich die Dichtelementaufnahme mit dem Dichtelement in der Position mit maximalem Abstand zum Fluidanschluss befindet.

Demnach kann hinsichtlich der vorstehenden Reduzierung des Risikos einer unbeabsichtigten Bewegung das Austreten eines Fluids in einem dekonnektierten Zustand verhindert werden.

In einer Ausgestaltung ist der Kupplungsgehäusegewindeabschnitt als Innengewinde ausgebildet und die Dichtelementaufnahmeführungsstruktur weist zumindest einen in das Innengewinde eingreifbaren in Bezug auf die Längsachse radial nach außen weisenden Vorsprung auf.

Die Dichtelementaufnahmeführungsstruktur kann somit in einfacher Weise ausgebildet werden. Grundsätzlich ist es aber auch möglich, den Kupplungsgehäusegewindeabschnitt als Außengewinde auszubilden, wobei die Dichtelementaufnahmeführungsstruktur zumindest abschnittsweise als hierzu korrespondierendes Innengewinde ausgestaltet ist.

Insbesondere weist der Kupplungsgehäusegewindeabschnitt zumindest zwei voneinander getrennte, insbesondere zumindest zwei einander gegenüberliegende Gewindegänge aufweist.

In Verbindung mit zumindest zwei entsprechend einander gegenüberliegenden Dichtelementaufnahmeführungsstrukturen kann die Dichtelementaufnahme im Kupplungsgehäusegewindeabschnitt positionsstabiler geführt werden, da hierdurch ein Verkippen der Dichtelementaufnahme verhindert wird. Die Anzahl der Gewindegänge kann jedoch auch geringer sein als die Anzahl der Dichtelementaufnahmeführungsstrukturen, um beispielsweise die Dichtelementaufnahme in unterschiedlichen Orientierung in das Kupplungsgehäuse einzusetzen und/oder unterschiedliche Bewegungen der Dichtelementaufnahme durch unterschiedliche Gewindegänge vorzusehen.

In einer Ausgestaltung erstreckt sich zumindest ein Gewindegang des Kupplungsgehäusegewindeabschnitts in Bezug auf die Längsachse über einen Winkel kleiner 360°, insbesondere über einen Winkel von im Wesentlichen 180°.

Die geführte Bewegung der Dichtelementaufnahme kann somit über einen begrenzten Drehwinkel erfolgen, was die Handhabung vereinfacht. Zudem erleichtert ein verkürzter Drehwinkel, also ein Drehwinkel zur Erreichung einer Zielposition, der nicht mehrere Umdrehungen umfasst, die Kontrolle der Bewegung, da nicht mehrere Umdrehungen nachverfolgt werden müssen. In diesem Zusammenhang können optische oder haptische Markierungselemente die Bewegungskontrolle unterstützen.

In einer Weiterbildung weist zumindest ein Gewindegang des Kupplungsgehäusegewindeabschnitt an seinem dem Fluidanschluss zugewandten Ende einen zur Längsachse orthogonalen Abschnitt aufweist.

Die Steigung des Gewindegangs wird somit über diesen Abschnitt nicht fortgesetzt. Wenn die Dichtelementaufnahmeführungsstruktur in den orthogonalen Abschnitt bewegt wird, kann die Dichtelementaufnahme nicht durch Aufbringung reiner Zug- oder Druckkräfte in Richtung der Längsachse aus dieser Position bewegt werden. Entsprechend ermöglicht der orthogonale Abschnitt eine Sicherung der Positionierung der Dichtelementaufnahme in dieser Position, insbesondere in der Position mit minimalem Abstand zum Fluidanschluss, wenn der orthogonale Abschnitt zu dieser Position korrespondiert.

Insbesondere weist zumindest ein Gewindegang des Kupplungsgehäusegewindeabschnitts an seinem dem Fluidanschluss zugewandten Ende einen in Richtung der Längsachse rückversetzten Abschnitt auf, der gegenüber dem Gewindegang in Richtung der Kupplungsseite rückversetzt ist.

Wenn die Dichtelementaufnahmeführungsstruktur in den rückversetzen Abschnitt bewegt wird, kann die Dichtelementaufnahme nicht durch Aufbringung reiner Drehbewegungen um die Längsachse aus dieser Position bewegt werden, sondern muss beispielweise zunächst durch Aufbringung einer Druckkraft auf die Dichtelementaufnahme entlang der Längsachse in Richtung des Fluidanschlusses aus dem rückversetzten Abschnitt bewegt werden. Die Positionierung der Dichtelementaufnahmeführungsstruktur im rückversetzten Abschnitt entspricht bevorzugt einer Position der Dichtelementaufnahme im konnektierten Zustand. Der rückversetzte Abschnitt kann alternativ oder ergänzend zum orthogonalen Abschnitt vorgesehen werden. Bei einer Kombination des rückversetzen und orthogonalen Abschnitts schließt sich der rückversetzte Abschnitt bevorzugt in Führungsrichtung der Dichtelementaufnahmeführungsstruktur an einem dem Fluidanschluss zugewandten Ende des Gewindegangs an den orthogonalen Abschnitt an. Beispielsweise wird die Dichtelementaufnahmeführungsstruktur zunächst entlang des zumindest einen eine Steigung aufweisenden Gewindegangs in Richtung des Fluidanschlusses bewegt, erreicht dann den orthogonalen Abschnitt und kann dann durch eine fortgesetzte Drehbewegung in den rückversetzten Abschnitt bewegt werden.

In einer Ausgestaltung ist der Gehäuseabschnitt relativ zur Längsachse drehbar.

Die Dichtelementaufnahme kann somit über eine Drehbewegung des Gehäuseabschnitts um die Längsachse über die Dichtelementaufnahmeführungsstruktur entlang der Längsachse bewegt werden. Bevorzugt ist der Gehäuseabschnitt dabei ein gegenüber dem übrigen Gehäuse drehbar gelagerter Abschnitt, so dass die durch den Gehäuseabschnitt ausgeführte Drehbewegung nicht auf das übrige Gehäuse übertragen wird. Dies erleichtert die Handhabung bzw. Konnektierung und Dekonnektierung insbesondere, wenn beispielsweise weitere Komponenten bereits an den Fluidanschluss angeschlossen sind.

Alternativ oder ergänzend ist die Dichtelementaufnahme relativ zur Längsachse und/oder zum Kupplungselementgehäuse drehbar.

Demnach wird nicht oder nicht nur der Gehäuseabschnitt zur Bewegung der Dichtelementaufnahme über die Dichtelementaufnahmeführungsstruktur im Eingriff mit zumindest einem entsprechenden Gewindegang gedreht, sondern die Dichtelementaufnahme.

Gemäß einer Weiterbildung bildet die Dichtelementaufnahme zusammen mit dem Dichtelement zumindest einen Teil einer kupplungsseitigen Stirnfläche des Kupplungselements aus.

Die Dichtelementaufnahme und das Dichtelement sind somit nicht rückversetzt, so dass sie eine gute Zugänglichkeit aufweisen, wodurch eine Desinfektion erleichtert wird. Die kupplungsseitige Stirnfläche kann insbesondere im Wesentlichen plan ausgeführt werden. Der Begriff "im Wesentlichen plan" ist darauf gerichtet, dass kleinere Konturabweichungen, wie beispielsweise eine mittige Überhöhung durch das Dichtelement, ausgebildet sein können, diese aber keinen Einfluss auf die kupplungsseitige Stirnfläche des Kupplungselements im Sinne einer Zugänglichkeit haben.

Gemäß einer Weiterbildung ist die Dichtelementaufnahme über ein elastisches Element, insbesondere ein Druckfederelement, das in Richtung der Längsachse wirkt und zwischen dem Fluidanschluss und der Dichtelementaufnahme angeordnet ist, im Kupplungselementgehäuse gelagert.

Das elastische Element kann über ein separates Federelement oder durch das Dichtelement oder die Dichtelementaufnahme gebildet werden. Sofern das elastische Element durch das Dichtelement oder die Dichtelementaufnahme gebildet wird, ist dieses Element in die entsprechenden Komponenten integriert oder wird durch diese, beispielsweise durch entsprechende Materialauswahl und/oder Strukturausbildung, ausgebildet. Das Dichtelement oder die Dichtelementaufnahme stützt sich in diesem Fall, ebenso wie ein separates Federelement in Längsrichtung gegen das Kupplungselementgehäuse ab. Die Dichtelementaufnahme wird über das elastische Element ohne äußere Krafteinwirkung in einer Position mit maximalem Abstand zum Fluidanschluss gehalten. Entsprechend erfordert eine Bewegung der Dichtelementaufnahme in Richtung des Fluidanschlusses eine Überwindung der Haltekraft, so dass das Risiko einer unbeabsichtigten Bewegung vermindert wird. Zudem unterstützt das elastische Element eine Rückbewegung von einem konnektierten Zustand in einen dekonnektierten Zustand.

In einem weiteren Aspekt ist die Erfindung auch auf ein Kupplungssystem für ein geschlossenes Fluidtransfersystem gerichtet. Das Kupplungssystem umfasst zumindest ein vorstehend beschriebenes Kupplungselement sowie zumindest ein Gegenkupplungselement zur Kupplung mit dem Kupplungselement. Das Gegenkupplungselement umfasst ein Gegenkupplungselementgehäuse mit einem Gegenkupplungselementfluidanschluss und einer Gegenkupplungsseite, wobei das Gegenkupplungselementgehäuse eine sich von dem Gegenkupplungselementfluidanschluss in Richtung der Gegenkupplungsseite erstreckende Gegenkupplungselementlängsachse aufweist, sowie ein Gegenkupplungselementdichtelement, das in dem Gegenkupplungselementgehäuse angeordnet ist und zusammen mit dem Gegenkupplungselementgehäuse zumindest einen Teil einer gegenkupplungsseitigen Stirnfläche des Gegenkupplungselements ausbildet. Das Kupplungssystem ist derart konfiguriert, dass die Fluidöffnung des Kupplungselements in einem mit dem Gegenkupplungselement konnektierten Zustand, in dem sich die Dichtelementaufnahme in der Position mit minimalem Abstand zum Fluidanschluss des Kupplungselements befindet, zumindest teilweise auf einer dem Gegenkupplungselementfluidanschluss zugewandten Seite des Gegenkupplungselementdichtelement des Gegenkupplungselements angeordnet ist.

Durch die Bewegung der Dichtelementaufnahme von einer Position mit maximalem Abstand zum Fluidanschluss in eine Position mit minimalem Abstand zum Fluidanschluss und entsprechender Bewegung des Gegenkupplungselements, wobei die gegenkupplungsseitige Stirnfläche des Gegenkupplungselementdichtelements und die kupplungsseitige Stirnfläche des Dichtelements des Kupplungselements zumindest im Bereich des Dorns fluiddicht aneinanderliegen, werden das Dichtelement des Kupplungselements und das Gegenkupplungselementdichtelement in Längsrichtung entlang des Dorns 30 in Richtung des Fluidanschlusses bewegt. Für eine fluide Verbindung des Kupplungselements mit dem Gegenkupplungselement über den Dorn ist das Kupplungssystem derart konfiguriert, dass das Dichtelement und das Gegenkupplungselementdichtelement soweit in Richtung des Fluidanschlusses des Kupplungselements bewegt werden, dass die Fluidöffnung des Dorns des Kupplungssystems in einem konnektierten Zustand, in dem sich die Dichtelementaufnahme in der Position mit minimalem Abstand zum Fluidanschluss des Kupplungselements befindet, zumindest teilweise, insbesondere vollständig auf einer der gegenkupplungselementseitigen Stirnfläche abgewandten Seite des Gegenkupplungsdichtelements in einen durch das Gegenkupplungselementgehäuse umfassten oder gebildeten Gegenkupplungselementfluidkanal ragt. Entsprechend wird die Fluidöffnung bis kurz vor Erreichen des konnektierten Zustands über das Dichtelement und das Gegenkupplungsdichtelement durchgängig abgedichtet. Werden das Kupplungselement und das Gegenkupplungselement dekonnektiert, kann gegebenenfalls noch am Dorn befindliches Fluid am Gegenkupplungselementdichtelement abgestreift werden, so dass das Risiko eines Austritts eines Fluids bei oder nach der Dekonnektierung verringert wird.

In einer Ausgestaltung weist das Gegenkupplungselementgehäuse einen Befestigungsabschnitt auf, der sich von der Gegenkupplungsseite in Richtung der Gegenkupplungselementlängsachse in Richtung des Gegenkupplungselementfluidanschlusses erstreckt und eine gegenkupplungselementseitige Befestigungsstruktur, insbesondere eine Aussparung, zur Verbindung mit der kupplungselementseitigen Befestigungsstruktur, insbesondere zur Aufnahme der Schnapphaken, umfasst.

Die gegenkupplungselementseitige Befestigungsstruktur in somit von der Gegenkupplungsseite aus gesehen gegenüber der gegenkupplungsseitigen Stirnfläche rückversetzt, wodurch die gegenkupplungsseitige Stirnfläche des Gegenkupplungselementdichtelements mit vorbestimmter Flächenpressung bei Befestigung des Gegenkupplungselements mit dem Kupplungselement gehalten werden kann. Gleichzeitig wird die Zugänglichkeit der gegenkupplungsseitigen Stirnfläche, beispielsweise zu Desinfektionszwecken, hierdurch nicht beschränkt.

Die gegenkupplungsseitige Befestigungsstruktur kann in Bezug auf die Gegenkupplungselementlängsachse radial umlaufend durchgängig sein, um eine Befestigung des Gegenkupplungselements mit dem Kupplungselement in beliebigen radialen Relativposition zu ermöglichen. Alternativ kann die gegenkupplungselementseitige Befestigungsstruktur nur abschnittsweise ausgebildet sein, um eine entsprechende Befestigung nur in einer oder mehreren vorbestimmten Relativpositionen zu erlauben. Beispielsweise kann bei nur einer zur Befestigung vorgesehenen Relativposition die gegenkupplungselementseitige Befestigungsstruktur Aussparungen aufweisen, die in ihrer Position und Dimensionierung in eineindeutiger Weise zu den Schnapphaken eines Kupplungselements korrespondieren.

In einer Ausgestaltung weist das Gegenkupplungselementgehäuse an einer sich in Richtung der Gegenkupplungselementlängsachse erstreckenden Außenwand einen Entriegelungsmechanismus auf, über den die Verbindung der gegenkupplungselementseitigen Befestigungsstruktur mit der kupplungselementseitigen Befestigungsstruktur lösbar ist.

Ein solcher Entriegelungsmechanismus kann beispielsweise über einen elastischen oder elastisch gelagerter Gegenkupplungselementgehäuseabschnitt ausgebildet werden, der einen Teil der gegenkupplungselementseitigen Befestigungsstruktur umfasst oder damit zusammenwirkt, so dass dieser derart beweglich ist, dass eine kupplungselementseitige Befestigungsstruktur aus der gegenkupplungselementseitigen Befestigungsstruktur bewegbar ist. In diesem Zusammenhang ist es vorteilhaft, wenn das Kupplungselementgehäuse derart ausgebildet ist, dass eine solche Entriegelung in einem Zustand, in dem sich die Dichtelementaufnahme in einer Position mit minimalem Abstand zum Fluidanschluss befindet, unterbunden wird.

Weitere Vorteile des Kupplungssystems ergeben sich zudem analog zu den zum Kupplungselement und/oder dem Gegenkupplungselement angeführten Vorteilen.

Merkmale, Zweckmässigkeiten und Vorteile der Erfindung werden nachfolgend auch anhand von Ausführungsbeispielen unter Bezugnahme auf die Zeichnungen beschrieben.

Es zeigt
Fig. 1 eine schematische Querschnittsansicht eines Kupplungselements in einer Ebene parallel zur Längsachse des Kupplungselementgehäuses gemäß einer exemplarischen ersten Ausführungsform des Kupplungselements im dekonnektierten Zustand;
Fig. 2 einen Ausschnitt eines exemplarischen Endes eines fluidanschlussseitigen Gewindegangs mit einem orthogonalen Abschnitt in einer Draufsicht in Blickrichtung auf die Längsachse;
Fig. 3 einen Ausschnitt eines weiteren exemplarischen Endes eines fluidanschlussseitigen Gewindegangs mit einem rückversetzten Abschnitt in einer Draufsicht in Blickrichtung auf die Längsachse;
Fig. 4 eine schematische Querschnittsansicht eines Gegenkupplungselements in einer Ebene parallel zur Gegenkupplungselementlängsachse des Gegenkupplungselementgehäuses gemäß einer exemplarischen ersten Ausführungsform des Gegenkupplungselements im dekonnektierten Zustand;
Fig. 5 eine schematische Querschnittsansicht eines Kupplungssystems mit einem Kupplungselement gemäß Fig. 1 und einem Gegenkupplungselement gemäß Fig. 4 in einer Ebene parallel zur Längsachse im dekonnektierten Zustand, in dem das Gegenkupplungselement durch das Kupplungselement gehalten wird;
Fig. 6 eine schematische Querschnittsansicht eines Kupplungssystems gemäß Fig. 5 in einer Ebene parallel zur Längsachse im konnektierten Zustand;
Fig. 7 eine Übersicht aller Außenansichten des Kupplungssystems gemäß den Figuren 4 und 5, der Schnittansichten entlang der Schnittlinie A-A und Schnittlinie B-B sowie eine perspektivische Ansicht im dekonnektierten Zustand;
Fig. 8 eine Übersicht aller Außenansichten des Kupplungssystems gemäß Fig. 7 bzw. den Figuren 4 und 5, der Schnittansichten entlang der Schnittlinie A-A und Schnittlinie B-B sowie eine perspektivische Ansicht in einem dekonnektierten Zustand, in dem das Gegenkupplungselement durch das Kupplungselement gehalten wird;
Fig. 9 eine Übersicht aller Außenansichten des Kupplungssystems gemäß den Figuren 7 und 8 bzw. den Figuren 4 und 5, der Schnittansichten entlang der Schnittlinie A-A und Schnittlinie B-B sowie eine perspektivische Ansicht im konnektierten Zustand.

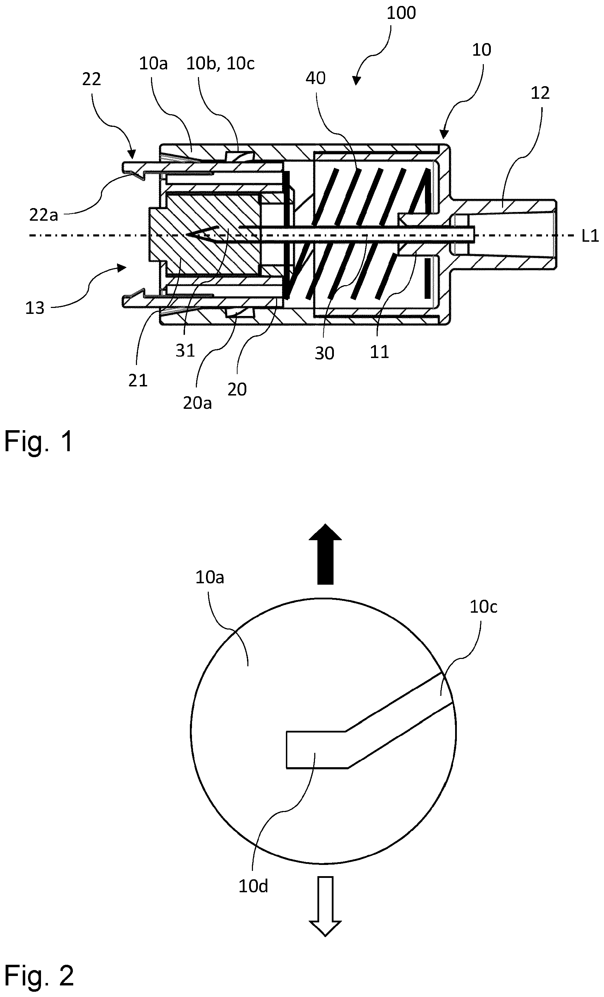

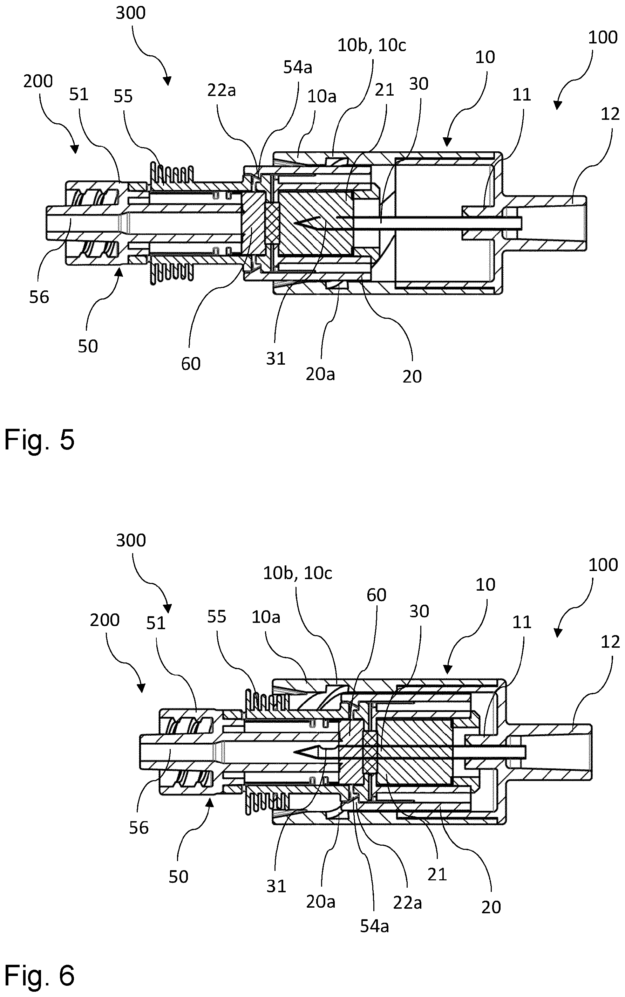

Fig. 1 zeigt eine Querschnittsansicht eines Kupplungselements 100 in einer Ebene parallel zur Längsachse L1 eines Kupplungselementgehäuses 10 des Kupplungselements 100 in einer exemplarischen ersten Ausführungsform. Die Schnittlinie korrespondiert zu der in Figur 6 gezeigten Schnittlinie A-A des Kupplungssystems 300. Die Längsachse L1 des Kupplungselements 100 verläuft von einem Fluidanschluss 12 in Richtung einer Kupplungsseite 13. Das Kupplungselement 100 umfasst neben dem Kupplungselementgehäuse 10 eine Dornaufnahme 11, einen in der Dornaufnahme 11 aufgenommenen Dorn 30, der als Fluidkanal ausgebildet ist, eine Dichtelementaufnahme 20 mit einer Dichtelementaufnahmeführungsstruktur 20a, ein in der Dichtelementaufnahme 20 aufgenommenes Dichtelement 21 sowie ein elastisches Element 40, das zwischen einen fluidanschlussseitigen Ende des Kupplungselementgehäuses 10 und der Dichtelementaufnahme 20 angeordnet ist.

Das Kupplungselementgehäuse 10 weist einen Gehäuseabschnitt 10a auf, der sich von dem fluidanschlussseitigen Abschnitt des Kupplungselementgehäuses 10 in Richtung der Kupplungsseite 13 um die Längsachse L1 erstreckt. In der gezeigten Ausführungsform ist der Gehäuseabschnitt 10a bevorzugt um die Längsachse L1 drehbar gelagert. Der Gehäuseabschnitt 10a umfasst zudem einen Kupplungsgehäusegewindeabschnitt 10b mit zwei Gewindegängen 10c, wobei jedoch auch mehr oder weniger Gewindegänge 10c vorgesehen sein können. Die Gewindegänge 10c sind hier jeweils als von der Kupplungsseite 13 in Richtung des Fluidanschlusses 12 über einen Winkel von 180° wendelförmig verlaufende Nuten ausgebildet. Ein kupplungsseitiges Ende des einen Gewindegangs 10c liegt dabei einem kupplungsseitigen Ende des anderen Gewindegangs 10c in Bezug auf die Längsachse L1 gegenüber. Entsprechend liegen sich auch die jeweiligen fluidanschlussseitigen Enden der Gewindegänge 10c in Bezug auf die Längsachse L1 gegenüber.

Im dargestellten dekonnektierten Zustand befindet sich die Dichtelementaufnahme 20 in einer Position mit maximalem Abstand zum Fluidanschluss 12 und wird über zwei Dichtelementaufnahmeführungsstrukturen 20a, die jeweils in einen der Gewindegänge 10c eingreifen im Kupplungselementgehäuse 10 gehalten. Die vorzusehenden Dichtelementaufnahmeführungsstrukturen 20a dienen primär der geführten Bewegung der Dichtelementaufnahme 20 entlang der Längsachse L1 von einer Position der Dichtelementaufnahme 20 von einer Position mit maximalem Abstand zum Fluidanschluss 12 zu einer Position mit minimalem Abstand zum Fluidanschluss 12 und umgekehrt. Demnach kann das Dichtelement 20 auch durch andere Strukturausgestaltungen im Kupplungselementgehäuse 10 gehalten werden und die jeweiligen Dichtelementaufnahmeführungsstrukturen 20a greifen erst mit Bewegung der Dichtelementaufnahme 20 entlang der Längsachse L1 in Richtung des Fluidanschlusses 12 in die jeweiligen Gewindegänge 10c vollständig ein.

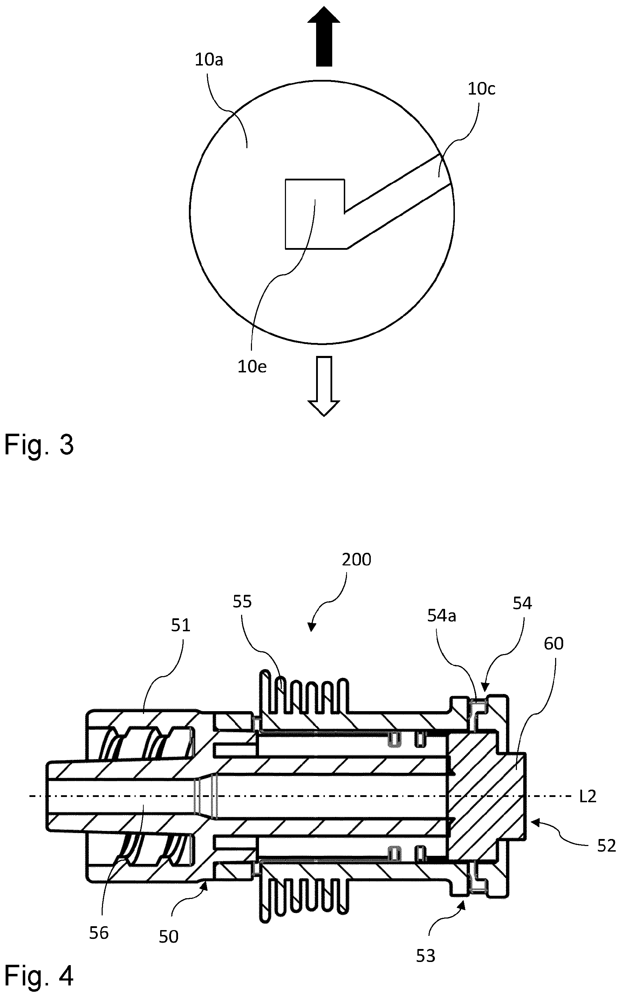

Zudem weist die Dichtelementaufnahme 20 an ihrem der Kupplungsseite 13 zugewandten Ende beispielhaft zwei kupplungselementseitige Befestigungsstrukturen 22 mit an den kupplungsseitigen Enden ausgebildeten Schnapphaken 22a auf. Über die Befestigungsstrukturen 22 mit den Schnapphaken 22a kann ein später noch beschriebenes Gegenkupplungselement 200, wie beispielsweise in Fig. 4 gezeigt, in vorbestimmter Positionsbeziehung zum Kupplungselement 100 gehalten werden. Die Befestigungsstruktur gemäß Fig. 1 erlaubt eine gute Zugänglichkeit zu der durch die Dichtelementaufnahme 20 und das Dichtelement 21 gebildete kupplungsseitige Stirnfläche, so dass dieser ohne wesentliche Einschränkungen desinfizierbar ist.

Das Dichtelement 21 ist derart in der Dichtelementaufnahme 20 angeordnet und dimensioniert, dass es in einem dekonnektierten Zustand eine in einem der Kupplungsseite 13 zugewandten Endabschnitt des Dorns 30 angeordnete Fluidöffnung 31 vollständig umgibt, also abdichtet.

Die Bewegung der Dichtelementaufnahme 20 von einer Position mit maximalem Abstand zum Fluidanschluss 12 in Richtung einer Position mit minimalem Abstand zum Fluidanschluss 12 oder umgekehrt erfolgt über die Führung der jeweiligen Dichtelementaufnahmeführungsstrukturen 20a in den jeweiligen Gewindegängen 10c in Richtung des Fluidanschlusses 12 oder entgegengesetzt hierzu in Abhängigkeit der vorzusehenden Bewegungsrichtung. Hierzu kann der Gehäuseabschnitt 10a und/oder die Dichtelementaufnahme 20 gezielt in eine Drehbewegung versetzt werden. Dies entspricht einem gezielten Ein- oder Ausschrauben. Alternativ oder ergänzend kann jedoch die Aufbringung einer Druck- oder Zugkraft in Abhängigkeit der vorzusehenden Bewegungsrichtung ausreichend sein, wenn der Gehäuseabschnitt 10a und/oder die Dichtelementaufnahme 20 derart drehbar gelagert sind, dass sich hierdurch der Gehäuseabschnitt 10a und/oder die Dichtelementaufnahme 20 eigenständig ein- oder ausschraubt.

Sofern ein eigenständiges Ein- oder Ausschrauben grundsätzlich möglich ist oder auch aus anderen Sicherungsüberlegungen heraus, kann es vorteilhaft sein, eine Position der jeweiligen Dichtelementaufnahmeführungsstrukturen 20a in den jeweiligen Gewindegängen 10c in einer Position, die zu einer Position mit minimalem Abstand zum Fluidanschluss 12 korrespondiert, gegen eine unbeabsichtigte Positionsveränderung zu sichern. Mögliche beispielhafte Ausgestaltungen zu einer solchen Positionssicherung sind in den Figuren 2 und 3 dargestellt, die jeweils einen Ausschnitt eines Endes eines fluidanschlussseitigen Gewindegangs 10c mit einem in einer Draufsicht in Blickrichtung auf die Längsachse L1 zeigen. Der ausgefüllte Pfeil repräsentiert dabei jeweils eine Längsachsenrichtung in Richtung der Kupplungsseite 13, während der nichtausgefüllte Pfeil eine Längsachsenrichtung in Richtung des Fluidanschlusses 12 repräsentiert.

Fig. 2 veranschaulicht ein exemplarisches Ende eines fluidanschlussseitigen Gewindegangs 10c mit einem zur Längsachse L1 orthogonalen Abschnitt 10d. Wird eine Dichtelementaufnahmeführungsstruktur 20a in den orthogonalen Abschnitt 10d bewegt, so bewirkt eine aufgebrachte Druck- oder Zugkraft keine Drehbewegung des Gehäuseabschnitts 10a und/oder der Dichtelementaufnahme 20. Entsprechend wird das Risiko einer unbeabsichtigten Bewegung verringert.

Alternativ zeigt Fig. 3 ein exemplarisches Ende eines fluidanschlussseitigen Gewindegangs 10c mit einem in Richtung des ausgefüllten Pfeils rückversetzten Abschnitt 10e. Wird eine Dichtelementaufnahmeführungsstruktur 20a in den rückversetzten Abschnitt 10e bewegt, so wird die Position der Dichtelementaufnahme 20 hierüber gegenüber Drehbewegungen gesichert. Diese Position der Dichtelementaufnahmeführungsstruktur 20a korrespondiert zu einer Position der Dichtelementaufnahme 20 in einem konnektierten Zustand, in dem sich die Dichtelementaufnahme 20 in der Position mit minimalem Abstand zum Fluidanschluss 12 des Kupplungselements 100 befindet. Streng genommen wird die Position der Dichtelementaufnahme 20 mit minimalem Abstand zum Fluidanschluss 12 durch die Position des Übergangs zwischen dem noch eine Steigung aufweisenden Abschnitt des Gewindegangs 10c und dem rückversetzten Abschnitt 10e gebildet. Da der Rückversatz im Anwendungsmaßstab jedoch unerheblich ist, wird auch die Position der Dichtelementaufnahme 20, wenn die Dichtelementführungsstruktur 20a in den rückversetzten Abschnitt 10e eingreift, als Position mit minimalem Abstand zum Fluidanschluss 12 verstanden.

Zur Lösung der Positionssicherung über den rückversetzten Abschnitt 10e muss die Dichtelementaufnahmeführungsstruktur 20a zunächst wieder relativ in Richtung des Fluidanschlusses 12 bewegt werden, um den rückversetzten Abschnitt 10e überwinden zu können. Diese Form der Positionssicherung wird zudem über die Vorsehung des elastischen Elements 40 unterstützt. Die Positionssicherung gemäß Fig. 3 schließt aber eine Positionssicherung gemäß Fig. 2 nicht aus, so dass auch kombinierte Positionssicherungen anwendbar sein können.

Fig. 4 zeigt eine schematische Querschnittsansicht eines Gegenkupplungselements 200 in einer Ebene parallel zur einer Gegenkupplungselementlängsachse L2 eines Gegenkupplungselementgehäuses 50 gemäß einer exemplarischen ersten Ausführungsform des Gegenkupplungselements 200 im dekonnektierten Zustand. Das Gegenkupplungselement 200 umfasst das Gegenkupplungselementgehäuse 50 mit einem Gegenkupplungselementfluidanschluss 51 und einer Gegenkupplungsseite 52, wobei das Gegenkupplungselementgehäuse 50 die sich von dem Gegenkupplungselementfluidanschluss 51 in Richtung der Gegenkupplungsseite 52 erstreckende Gegenkupplungselementlängsachse L2 aufweist. Zudem umfasst das Gegenkupplungselement ein Dichtelement 60, das an einem der Gegenkupplungsseite 52 zugewandten Ende des Gegenkupplungselements 200 im Gegenkupplungselementgehäuse 50 angeordnet ist und zusammen mit dem Gegenkupplungselementgehäuse 50 eine gegenkupplungsseitige Stirnfläche des Gegenkupplungselements 200 ausbildet. Das Gegenkupplungselementdichtelement 60 dichtet einen sich vom Gegenkupplungselementfluidanschluss 51 in Richtung der Gegenkupplungsseite 52 erstreckenden Gegenkupplungselementfluidkanal 56, der hier exemplarisch koaxial zur Gegenkupplungselementlängsachse L2 verläuft, gegenkupplungsseitig ab.

Das Gegenkupplungselementgehäuse 50 weist einen Befestigungsabschnitt 53 auf, der sich von der Gegenkupplungsseite 52 in Richtung der Gegenkupplungselementlängsachse L2 in Richtung des Gegenkupplungselementfluidanschlusses 51 erstreckt und eine gegenkupplungselementseitige Befestigungsstruktur 54, hier beispielsweise zwei Aussparungen 54a, zur Verbindung mit der kupplungselementseitigen Befestigungsstruktur 22, umfasst. Die Aussparungen 54a sind in der gezeigten Ausführungsform nur abschnittsweise gebildet und korrespondieren zur Position und Dimensionierung der Schnapphaken 22a der Befestigungsstrukturen 22 des in Kupplungselements 100 gemäß Fig. 1. Entsprechend kann das Gegenkupplungselement 200 hier nur in zwei in Bezug auf die Gegenkupplungselementlängsachse L2 mit dem Kupplungselement 100 nach Fig. 1 verbunden werden.

Zudem weist das Gegenkupplungselementgehäuse 50 an einer sich in Richtung der Gegenkupplungselementlängsachse L2 erstreckenden Außenwand 50a zwei Entriegelungsmechanismen 55 auf, über die die Verbindung der gegenkupplungselementseitigen Befestigungsstruktur 54 mit den kupplungselementseitigen Befestigungsstrukturen 22 des Kupplungselements 100 gemäß Fig. 1 lösbar ist. Die Entriegelungsmechanismen 55 sind in der dargestellten ausführungsform jeweils an der Außenwand 50a elastisch gelagerte Gehäuseabschnitte, bei denen durch einen radialen Druck von außen in Richtung der Gegenkupplungselementlängsachse L2 auf einen der Gegenkupplungsseite 52 abgewandten Abschnitt ein der Gegenkupplungsseite 52 zugewandter Abschnitt, der die Aussparung 54a umfasst, radial nach außen, also entgegengesetzt zur Gegenkupplungselementlängsachse L2, bewegt wird. Ein in die Aussparung 54a eingreifender Schnapphaken würde somit ebenfalls radial nach außen bewegt, so dass eine Verbindung gelöst werden kann.

Anhand der Figuren 5 und 6 wird im Folgenden das Zusammenwirken des Kupplungselements 100 mit einem Gegenkupplungselement 200 in einem Kupplungssystem 300 beschrieben.

Fig. 5 zeigt hierzu eine schematische Querschnittsansicht eines Kupplungssystems 300 mit einem Kupplungselement 100 gemäß Fig. 1 und einem Gegenkupplungselement 200 gemäß Fig. 4 in einer Ebene parallel zur Längsachse L1 bzw. Gegenkupplungselementlängsachse L2 im dekonnektierten Zustand. Aus Gründen der Übersichtlichkeit sind hier die jeweiligen Längsachsen L1 bzw. L2 sowie das elastische Element 40 nicht eingezeichnet. Hierzu wird auf die Figuren 1 und 4 verwiesen.

In dem dargestellten dekonnektierten Zustand befinden sich Schnapphaken 22a im Eingriff mit den Aussparungen 54a, so dass das Gegenkupplungselement 200 durch das Kupplungselement 100 gehalten wird. Das Kupplungssystem 300 weist dennoch einen dekonnektierten Zustand auf, da die Dichtelementaufnahme 20 in einer Position mit maximalem Abstand zum Fluidanschluss 12 angeordnet ist, die keine fluide Verbindung zwischen dem Kupplungselement 100 und Gegenkupplungselement 200 ermöglicht. Durch den Eingriff der Schnapphaken 22a in die Aussparungen 54a werden die einander zugewandten Stirnflächen des Dichtelements 21 und des Gegenkupplungsdichtelements 60 mit konstanter Flächenpressung fluiddicht gegeneinander gedrückt. Die parallel zur Längsachse L1 angeordnete Fluidöffnung 31 wird über das Dichtelement 21 abgedichtet.

Hierzu zeigt Fig. 6 eine schematische Querschnittsansicht eines Kupplungssystems 300 gemäß Fig. 5 in einer Ebene parallel zur Längsachse L1 bzw. Gegenkupplungselementlängsachse L2 im konnektierten Zustand. Auch hier sind aus Gründen der Übersichtlichkeit die jeweiligen Längsachsen L1 bzw. L2 sowie das elastische Element 40 nicht eingezeichnet und es wird entsprechend auf die Figuren 1 und 4 verwiesen.

Durch Drehung der Dichtelementaufnahme 20, beispielsweise über Drehung des Gegenkupplungselements 100, in Richtung der Gewindegänge 10c bis zu einem fluidanschlussseitigen Ende der Gewindegänge 10c befindet sich die Dichtelementaufnahme 20 in einer Position mit minimalem Abstand zum Fluidanschluss 12. Diese Position entspricht einem konnektierten Zustand, in dem die Fluidöffnung 31 über die dem Gegenkupplungselementfluidanschluss 51 zugewandte Seite des Gegenkupplungselementdichtelements 60 in den Fluidkanal 60 hineinragt und somit eine fluide Verbindung ausbildet.

In dieser Ausführungsform ist der Gehäuseabschnitt 10a derart konfiguriert, dass die Schnapphaken 22a im konnektierten Zustand an einer der Längsachse L1 zugewandten Innenseite des Gehäuseabschnitts 10a anliegen. Hierüber wird die Verbindung des Gegenkupplungselements 200 mit dem Kupplungselement 100 gesichert, da diese über die Entriegelungsmechanismen 55 nicht gelöst werden können.

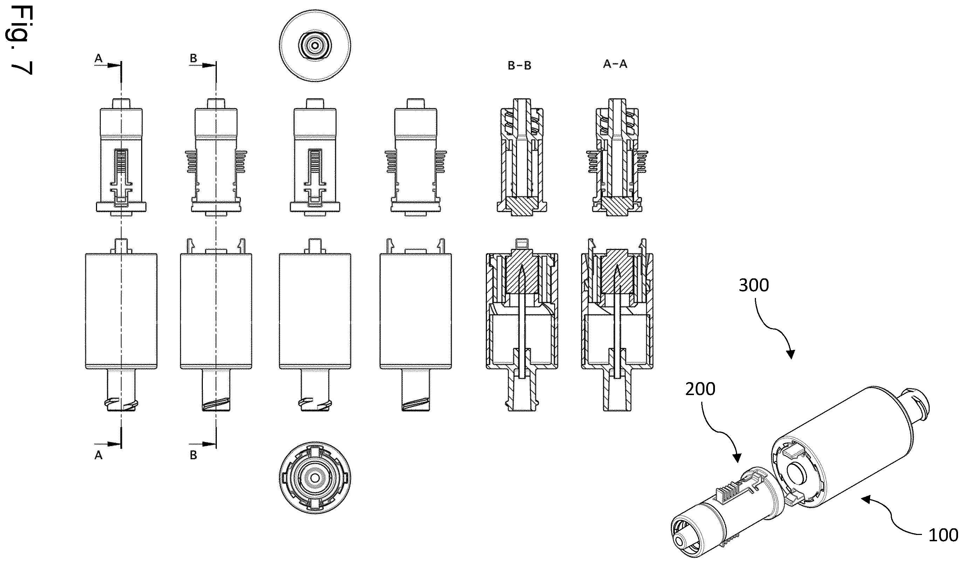

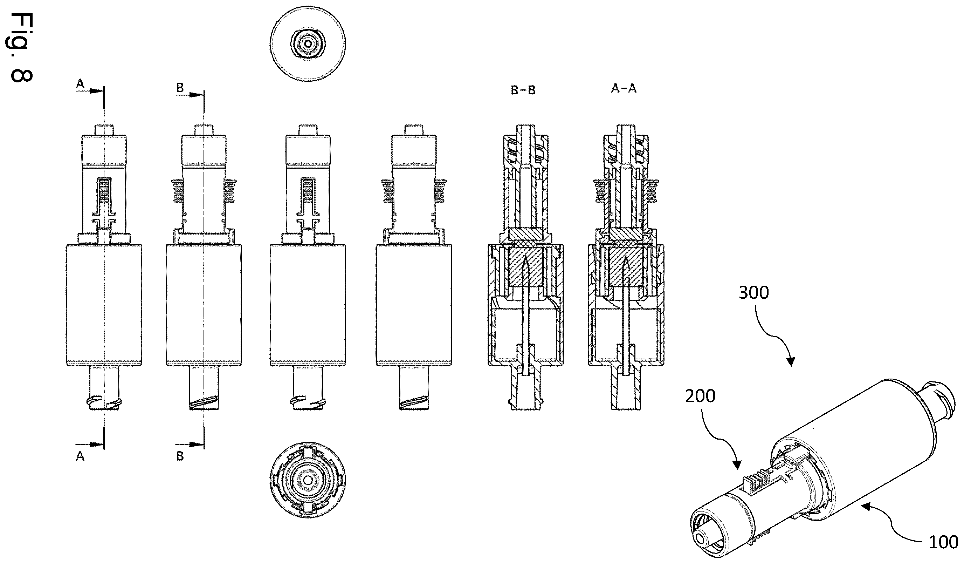

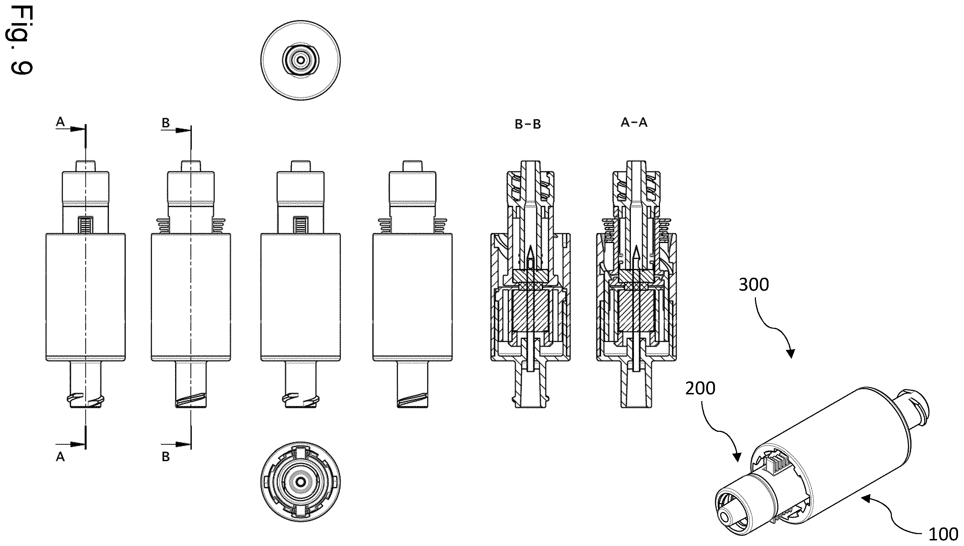

Fig. 7 zeigt ergänzend eine Übersicht aller Außenansichten des Kupplungssystems gemäß den Figuren 4 und 5, der Schnittansichten entlang der Schnittlinie A-A und Schnittlinie B-B sowie eine perspektivische Ansicht im dekonnektierten Zustand. Hieraus ergeben sich weitere Ausgestaltungsmerkmale der beschriebenen Ausführungsform. Analog hierzu zeigt Fig. 8 eine Übersicht des Kupplungssystems 300 gemäß Fig. 7 in einem dekonnektierten Zustand, in dem das Gegenkupplungselement 200 durch das Kupplungselement 100 gehalten wird, und Fig. 9 in einem konnektierten Zustand.

Die Erfindung ist nicht auf die beschriebenen Ausführungsformen beschränkt. Insbesondere sind bestimmte Merkmale einer Ausführungsform grundsätzlich auch auf andere Ausführungsformen anwendbar, sofern sich dies nicht vernünftigerweise ausschließt.

### Liste der Bezugszeichen:

- 10: Kupplungselementgehäuse
- 10a: Gehäuseabschnitt
- 10b: Kupplungsgehäusegewindeabschnitt
- 10c: Gewindegang
- 10d: orthogonaler Abschnitt (Gewindegang)
- 10e: rückversetzter Abschnitt (Gewindegang)
- 11: Dornaufnahme
- 12: Fluidanschluss
- 13: Kupplungsseite
- 20: Dichtelementaufnahme
- 20a: Dichtelementaufnahmeführungsstruktur
- 21: Dichtelement
- 22: kupplungselementseitige Befestigungsstruktur
- 22a: Schnapphaken
- 30: Dorn
- 31: Fluidöffnung
- 40: elastisches Element
- 50: Gegenkupplungselementgehäuse
- 51: Gegenkupplungselementfluidanschluss
- 52: Gegenkupplungsseite
- 53: Befestigungsabschnitt
- 54: gegenkupplungselementseitige Befestigungsstruktur
- 54a: Aussparung
- 55: Entriegelungsmechanismus
- 56: Gegenkupplungselementfluidkanal
- 60: Gegenkupplungselementdichtelement
- 100: Kupplungselement
- 200: Gegenkupplungselement
- 300: Kupplungssystem
- L1: Längsachse (Kupplungselement)
- L2: Gegenkupplungselementlängsachse

## Patentansprüche

1. Kupplungselement (100) für ein geschlossenes Fluidtransfersystem, umfassend:
ein Kupplungselementgehäuse (10) mit einem Fluidanschluss (12) und einer Kupplungsseite (13), wobei das Kupplungselementgehäuse (10) eine sich von dem Fluidanschluss (12) in Richtung der Kupplungsseite (13) erstreckende Längsachse (L1) aufweist,
einen Dorn (30) mit zumindest einer Fluidöffnung (31), der in einer am Fluidanschluss (12) angeordneten Dornaufnahme (11) des Gehäuses (10) gehalten wird und sich in Richtung der Längsachse (L1) in das Kupplungselementgehäuse (10) hinein erstreckt, wobei die zumindest eine Fluidöffnung (31) in einem der Kupplungsseite (13) zugewandten Endabschnitt des Dorns (30) angeordnet ist,
eine in dem Kupplungselementgehäuse (10) auf der Kupplungsseite angeordnete Dichtelementaufnahme (20) mit einer Dichtelementaufnahmeführungsstruktur (20a) und
ein Dichtelement (21), das in der Dichtelementaufnahme (20) angeordnet ist,
wobei das Kupplungsgehäuse (10) einen Gehäuseabschnitt (10a) aufweist, der die Dichtelementaufnahme (20) in Bezug auf die Längsachse (L1) in axialer Richtung zumindest abschnittsweise umgibt und auf der dem Dichtelement (21) zugewandten Innenseite einen Kupplungsgehäusegewindeabschnitt (10b) aufweist, und
wobei die Dichtelementaufnahme (20) mit dem Dichtelement (21) in Richtung der Längsachse (L1) über den Kupplungsgehäusegewindeabschnitt (10b) zwischen einer Position mit maximalem Abstand zum Fluidanschluss (12) und einer Position mit minimalem Abstand zum Fluidanschluss (12) über die Dichtelementaufnahmeführungsstruktur (20a) geführt bewegbar ist,
**dadurch gekennzeichnet, dass**
die Dichtelementaufnahme (20) an ihrem der Kupplungsseite (13) zugewandten Ende zumindest zwei in Richtung der Längsachse (L1) in Richtung der Kupplungsseite (13) vorstehende Schnapphaken (22a) als eine kupplungselementseitige Befestigungsstruktur (22) für ein Gegenkupplungselement (200) aufweist.

2. Kupplungselement (100) nach Anspruch 1, wobei die zumindest eine Fluidöffnung (31) im Dichtelement (21) angeordnet ist, wenn sich die Dichtelementaufnahme (20) mit dem Dichtelement (21) in der Position mit maximalem Abstand zum Fluidanschluss (12) befindet.

3. Kupplungselement (100) nach Anspruch 1 oder 2, wobei der Kupplungsgehäusegewindeabschnitt (10b) als Innengewinde ausgebildet ist und die Dichtelementaufnahmeführungsstruktur (20a) zumindest einen in das Innengewinde eingreifbaren in Bezug auf die Längsachse (L1) radial nach außen weisenden Vorsprung aufweist.

4. Kupplungselement (100) nach einen der vorherigen Ansprüche, wobei der Kupplungsgehäusegewindeabschnitt (10b) zumindest zwei voneinander getrennte, insbesondere zumindest zwei einander gegenüberliegende Gewindegänge (10c) aufweist.

5. Kupplungselement (100) nach einem der vorherigen Ansprüche, wobei sich zumindest ein Gewindegang (10c) des Kupplungsgehäusegewindeabschnitts (10b) in Bezug auf die Längsachse (L1) über einen Winkel kleiner 360°, insbesondere über einen Winkel von im Wesentlichen 180°, erstreckt.

6. Kupplungselement (100) nach einem der vorherigen Ansprüche, wobei zumindest ein Gewindegang (10c) des Kupplungsgehäusegewindeabschnitt (10b) an seinem dem Fluidanschluss (12) zugewandten Ende einen zur Längsachse (L1) orthogonalen Abschnitt (10d) aufweist.

7. Kupplungselement (100) nach einem der vorherigen Ansprüche, wobei zumindest ein Gewindegang (10c) des Kupplungsgehäusegewindeabschnitts (10b) an seinem dem Fluidanschluss zugewandten Ende einen in Richtung der Längsachse (L1) rückversetzten Abschnitt (10e) aufweist, der gegenüber dem Gewindegang (10c) in Richtung der Kupplungsseite (13) rückversetzt ist.

8. Kupplungselement (100) nach einem der vorherigen Ansprüche, wobei der Gehäuseabschnitt (10a) relativ zur Längsachse (L1) drehbar ist.

9. Kupplungselement (100) nach einem der vorherigen Ansprüche, wobei die Dichtelementaufnahme (20) relativ zur Längsachse (L1) und/oder zum Kupplungselementgehäuse (10) drehbar ist.

10. Kupplungselement (100) nach einem der vorherigen Ansprüche, wobei die Dichtelementaufnahme (20) zusammen mit dem Dichtelement (21) zumindest einen Teil einer kupplungsseitigen Stirnfläche des Kupplungselements (100) ausbildet.

11. Kupplungselement (100) nach einem der vorherigen Ansprüche, wobei die Dichtelementaufnahme (20) über ein elastisches Element (40), insbesondere ein Druckfederelement, das in Richtung der Längsachse wirkt und zwischen dem Fluidanschluss (12) und der Dichtelementaufnahme (20) angeordnet ist, im Kupplungselementgehäuse (10) gelagert ist.

12. Kupplungssystem (300) für ein geschlossenes Fluidtransfersystem, umfassend zumindest ein Kupplungselement (100) nach einem der Ansprüche 1 bis 11 sowie zumindest ein Gegenkupplungselement (200),
wobei das Gegenkupplungselement (200) umfasst:
ein Gegenkupplungselementgehäuse (50) mit einem Gegenkupplungselementfluidanschluss (51) und einer Gegenkupplungsseite (52), wobei das Gegenkupplungselementgehäuse (50) eine sich von dem Gegenkupplungselementfluidanschluss (51) in Richtung der Gegenkupplungsseite (52) erstreckende Gegenkupplungselementlängsachse (L2) aufweist, sowie
ein Gegenkupplungselementdichtelement (60), das in dem Gegenkupplungselementgehäuse (50) angeordnet ist und zusammen mit dem Gegenkupplungselementgehäuse (50) zumindest einen Teil einer gegenkupplungsseitigen Stirnfläche des Gegenkupplungselements (200) ausbildet;
wobei das Kupplungssystem (300) derart konfiguriert ist, dass die Fluidöffnung (31) des Kupplungselements (100) in einem mit dem Gegenkupplungselement (200) konnektierten Zustand, in dem sich die Dichtelementaufnahme (20) in der Position mit minimalem Abstand zum Fluidanschluss (12) des Kupplungselements (100) befindet, zumindest teilweise auf einer dem Gegenkupplungselementfluidanschluss (51) zugewandten Seite des Gegenkupplungselementdichtelement (60) des Gegenkupplungselements (200) angeordnet ist.

13. Kupplungssystem (300) nach Anspruch 12
wobei das Gegenkupplungselementgehäuse (50) einen Befestigungsabschnitt (53) aufweist, der sich von der Gegenkupplungsseite (52) in Richtung der Gegenkupplungselementlängsachse (L2) in Richtung des Gegenkupplungselementfluidanschlusses (51) erstreckt und eine gegenkupplungselementseitige Befestigungsstruktur (54), insbesondere eine Aussparung (54a), zur Verbindung mit der kupplungselementseitigen Befestigungsstruktur (22), insbesondere zur Aufnahme der Schnapphaken (22a), umfasst.

14. Kupplungssystem (300) nach Anspruch 13, wobei das Gegenkupplungselementgehäuse (50) an einer sich in Richtung der Gegenkupplungselementlängsachse (L2) erstreckenden Außenwand (50a) einen Entriegelungsmechanismus (55) aufweist, über den die Verbindung der gegenkupplungselementseitigen Befestigungsstruktur (54) mit der kupplungselementseitigen Befestigungsstruktur (22) lösbar ist.

## Claims

1. Coupling element (100) for a closed fluid transfer system, comprising:
a coupling element housing (10) with a fluid port (12) and a coupling side (13), wherein the coupling element housing (10) has a longitudinal axis (L1) extending from the fluid port (12) in the direction of the coupling side (13),
a pin (30) with at least one fluid opening (31), which is held in a pin receptacle (11) of the housing (10) arranged on the fluid port (12) and extends in the direction of the longitudinal axis (L1) into the coupling element housing (10), wherein the at least one fluid opening (31) is arranged in an end section of the pin (30) facing the coupling side (13),
a sealing element receptacle (20) with a sealing element receptacle guide structure (20a) and arranged in the coupling element housing (10) on the coupling side and
a sealing element (21), which is arranged in the sealing element receptacle (20),
wherein the coupling housing (10) has a housing section (10a), which surrounds the sealing element receptacle (20) in an axial direction at least in sections in relation to the longitudinal axis (L1) and has a coupling housing threaded section (10b) on the inside facing the sealing element (21), and
wherein the sealing element receptacle (20) can be moved with the sealing element (21) in the direction of the longitudinal axis (L1) via the coupling housing threaded section (10b) between a position with a maximum distance to the fluid port (12) and a position with a minimum distance to the fluid port (12) in a guided manner via the sealing element receptacle guide structure (20a),
**characterised in that**
the sealing element receptacle (20) has, at its end facing the coupling side (13), at least two snap hooks (22a) protruding in the direction of the longitudinal axis (L1) in the direction of the coupling side (13) as a fastening structure (22) on the coupling element side for a counter-coupling element (200).

2. Coupling element (100) according to claim 1, wherein the at least one fluid opening (31) is arranged in the sealing element (21) if the sealing element receptacle (20) with the sealing element (21) is in the position with the maximum distance from the fluid port (12).

3. Coupling element (100) according to claim 1 or 2, wherein the coupling housing threaded section (10b) is designed as an internal thread and the sealing element receptacle guide structure (20a) has at least one projection which can engage in the internal thread and points radially outwards in relation to the longitudinal axis (L1).

4. Coupling element (100) according to one of the preceding claims, wherein the coupling housing threaded section (10b) has at least two separate threads (10c), in particular at least two opposing threads (10c).

5. Coupling element (100) according to one of the preceding claims, wherein at least one thread (10c) of the coupling housing threaded section (10b) extends over an angle smaller than 360°, in particular over an angle of substantially 180°, in relation to the longitudinal axis (L1).

6. Coupling element (100) according to one of the preceding claims, wherein at least one thread (10c) of the coupling housing threaded section (10b) has a section (10d) orthogonal to the longitudinal axis (L1) at its end facing the fluid port (12).

7. Coupling element (100) according to one of the preceding claims, wherein at least one thread (10c) of the coupling housing threaded section (10b) has, at its end facing the fluid port, a section (10e) set back in the direction of the longitudinal axis (L1), which is set back in relation to the thread (10c) in the direction of the coupling side (13).

8. Coupling element (100) according to one of the preceding claims, wherein the housing section (10a) can be rotated relative to the longitudinal axis (L1).

9. Coupling element (100) according to one of the preceding claims, wherein the sealing element receptacle (20) can be rotated relative to the longitudinal axis (L1) and/or relative to the coupling element housing (10).

10. Coupling element (100) according to one of the preceding claims, wherein the sealing element receptacle (20) together with the sealing element (21) forms at least part of a coupling side end face of the coupling element (100).

11. Coupling element (100) according to one of the preceding claims, wherein the sealing element receptacle (20) is mounted in the coupling element housing (10) via an elastic element (40), in particular a compression spring element, which acts in the direction of the longitudinal axis and is arranged between the fluid port (12) and the sealing element receptacle (20).

12. Coupling system (300) for a closed fluid transfer system, comprising at least one coupling element (100) according to one of claims 1 to 11 and at least one counter-coupling element (200),
wherein the counter-coupling element (200) comprises:
a counter-coupling element housing (50) with a counter-coupling element fluid port (51) and a counter-coupling side (52), wherein the counter-coupling element housing (50) has a counter-coupling element longitudinal axis (L2) extending from the counter-coupling element fluid port (51) in the direction of the counter-coupling side (52), and
a counter-coupling element sealing element (60), which is arranged in the counter-coupling element housing (50) and together with the counter-coupling element housing (50) forms at least part of a counter-coupling side end face of the counter-coupling element (200);
wherein the coupling system (300) is configured in such a way that, in a state connected to the counter-coupling element (200) in which the sealing element receptacle (20) is in the position with a minimum distance to the fluid port (12) of the coupling element (100), the fluid opening (31) of the coupling element (100) is arranged at least partially on a side of the counter-coupling element sealing element (60) of the counter-coupling element (200) facing the counter-coupling element fluid port (31).

13. Coupling system (300) according to claim 12,
wherein the counter-coupling element housing (50) has a fastening section (53), which extends from the counter-coupling side (52) in the direction of the counter-coupling element longitudinal axis (L2) in the direction of the counter-coupling element fluid port (51) and comprises a counter-coupling element side fastening structure (54), in particular a recess (54a), for connection to the coupling element side fastening structure (22), in particular for receiving the snap hooks (22a).

14. Coupling system (300) according to claim 13, wherein the counter-coupling element housing (50) has, at an outer wall (50a) extending in the direction of the counter-coupling element longitudinal axis (L2), a release mechanism (55), via which the connection of the counter-coupling element side fastening structure (54) to the coupling element side fastening structure (22) can be released.

## Revendications

1. Élément d'accouplement (100) pour un système de transfert de fluide fermé, comprenant :
un boîtier d'élément d'accouplement (10) avec un raccord de fluide (12) et un côté accouplement (13), le boîtier d'élément d'accouplement (10) ayant un axe longitudinal (L1) s'étendant depuis le raccord de fluide (12) en direction du côté accouplement (13) ;
une broche (30) comportant au moins une ouverture de fluide (31), qui est maintenue dans un logement de broche (11) du boîtier (10) disposé sur le raccord de fluide (12) et qui s'étend dans le boîtier d'élément d'accouplement (10) dans la direction de l'axe longitudinal (L1), ladite au moins une ouverture de fluide (31) étant disposée dans une partie d'extrémité de la broche (30) faisant face au côté accouplement (13),
un logement d'élément d'étanchéité (20) disposé dans le boîtier d'élément d'accouplement (10) sur le côté accouplement et comportant une structure de guidage de logement d'élément d'étanchéité (20a) et
un élément d'étanchéité (21) qui est disposé dans le logement d'élément d'étanchéité (20),
dans lequel le boîtier d'accouplement (10) présente une partie de boîtier (10a) qui entoure au moins par sections le logement d'élément d'étanchéité (20) dans une direction axiale par rapport à l'axe longitudinal (L1) et qui présente, sur le côté intérieur faisant face à l'élément d'étanchéité (21), une partie filetée de boîtier d'accouplement (10b), et
dans lequel le logement d'élément d'étanchéité (20) peut être déplacé avec l'élément d'étanchéité (21) dans la direction de l'axe longitudinal (L1) par l'intermédiaire de la partie filetée de boîtier d'accouplement (10b) entre une position située à une distance maximale du raccord de fluide (12) et une position située à une distance minimale du raccord de fluide (12) de manière guidée par l'intermédiaire de la structure de guidage de logement d'élément d'étanchéité (20a), **caractérisé en ce que**
le logement d'élément d'étanchéité (20) présente, à son extrémité faisant face au côté accouplement (13), au moins deux crochets d'encliquetage (22a) faisant saillie dans la direction de l'axe longitudinal (L1) en direction du côté accouplement (13) en tant que structure de fixation côté élément d'accouplement (22) pour un élément d'accouplement complémentaire (200).

2. Élément d'accouplement (100) selon la revendication 1, dans lequel ladite au moins une ouverture de fluide (31) est disposée dans l'élément d'étanchéité (21) lorsque le logement d'élément d'étanchéité (20) avec l'élément d'étanchéité (21) se trouve dans la position présentant la distance maximale par rapport au raccord de fluide (12).

3. Élément d'accouplement (100) selon la revendication 1 ou 2, dans lequel la partie filetée de boîtier d'accouplement (10b) est conçue comme un filetage intérieur et la structure de guidage de logement d'élément d'étanchéité (20a) présente au moins une saillie pouvant s'engager dans le filetage intérieur et orientée radialement vers l'extérieur par rapport à l'axe longitudinal (L1).

4. Élément d'accouplement (100) selon l'une des revendications précédentes, dans lequel la partie filetée de boîtier d'accouplement (10b) présente au moins deux filets (10c) séparés l'un de l'autre, en particulier au moins deux filets (10c) opposés l'un à l'autre.

5. Élément d'accouplement (100) selon l'une des revendications précédentes, dans lequel au moins un filet (10c) de la partie filetée de boîtier d'accouplement (10b) s'étend sur un angle inférieur à 360°, en particulier sur un angle de sensiblement 180°, par rapport à l'axe longitudinal (L1).

6. Élément d'accouplement (100) selon l'une des revendications précédentes, dans lequel au moins un filet (10c) de la partie filetée de boîtier d'accouplement (10b) présente, à son extrémité faisant face au raccord de fluide (12), une partie (10d) orthogonale à l'axe longitudinal (L1).

7. Élément d'accouplement (100) selon l'une des revendications précédentes, dans lequel au moins un filet (10c) de la partie filetée de boîtier d'accouplement (10b) présente, à son extrémité faisant face au raccord de fluide, une partie (10e) en retrait dans la direction de l'axe longitudinal (L1), qui est en retrait en direction du côté accouplement (13) par rapport au filet (10c).

8. Élément d'accouplement (100) selon l'une des revendications précédentes, dans lequel la partie de boîtier (10a) peut tourner par rapport à l'axe longitudinal (L1).

9. Élément d'accouplement (100) selon l'une des revendications précédentes, dans lequel le logement d'élément d'étanchéité (20) peut tourner par rapport à l'axe longitudinal (L1) et/ou au boîtier d'élément d'accouplement (10).

10. Élément d'accouplement (100) selon l'une des revendications précédentes, dans lequel le logement d'élément d'étanchéité (20) forme, conjointement avec l'élément d'étanchéité (21), au moins une partie d'une surface frontale côté accouplement de l'élément d'accouplement (100).

11. Élément d'accouplement (100) selon l'une des revendications précédentes, dans lequel le logement d'élément d'étanchéité (20) est monté dans le boîtier d'élément d'accouplement (10) par l'intermédiaire d'un élément élastique (40), en particulier un élément ressort de compression, agissant dans la direction de l'axe longitudinal et disposé entre le raccord de fluide (12) et le logement d'élément d'étanchéité (20).

12. Système d'accouplement (300) pour un système de transfert de fluide fermé, comprenant au moins un élément d'accouplement (100) selon l'une des revendications 1 à 11, ainsi qu'au moins un élément d'accouplement complémentaire (200),
dans lequel l'élément d'accouplement complémentaire (200) comprend :
un boîtier d'élément d'accouplement complémentaire (50) avec un raccord de fluide d'élément d'accouplement complémentaire (51) et un côté accouplement complémentaire (52), le boîtier d'élément d'accouplement complémentaire (50) ayant un axe longitudinal d'élément d'accouplement complémentaire (L2) s'étendant depuis le raccord de fluide d'élément d'accouplement complémentaire (51) en direction du côté accouplement complémentaire (52), ainsi que
un élément d'étanchéité d'élément d'accouplement complémentaire (60) qui est disposé dans le boîtier d'élément d'accouplement complémentaire (50) et qui forme, conjointement avec le boîtier d'élément d'accouplement complémentaire (50), au moins une partie d'une surface frontale côté accouplement complémentaire de l'élément d'accouplement complémentaire (200) ;
dans lequel le système d'accouplement (300) est configuré de telle sorte que l'ouverture de fluide (31) de l'élément d'accouplement (100), dans un état connecté à l'élément d'accouplement complémentaire (200) dans lequel le logement d'élément d'étanchéité (20) se trouve dans la position présentant une distance minimale par rapport au raccord de fluide (12) de l'élément d'accouplement (100), est disposée au moins en partie sur un côté de l'élément d'étanchéité d'élément d'accouplement complémentaire (60) faisant face au le raccord de fluide d'élément d'accouplement complémentaire (51) de l'élément d'accouplement complémentaire (200).

13. Système d'accouplement (300) selon la revendication 12,
dans lequel le boîtier d'élément d'accouplement complémentaire (50) présente une partie de fixation (53) qui s'étend depuis le côté accouplement complémentaire (52) dans la direction de l'axe longitudinal d'élément d'accouplement complémentaire (L2) en direction du raccord de fluide d'élément d'accouplement complémentaire (51) et comprend une structure de fixation côté élément d'accouplement complémentaire (54), en particulier un évidement (54a), pour la liaison avec la structure de fixation côté élément d'accouplement (22), en particulier pour recevoir les crochets d'encliquetage (22a).

14. Système d'accouplement (300) selon la revendication 13, dans lequel le boîtier d'élément d'accouplement complémentaire (50) présente, sur une paroi extérieure (50a) s'étendant dans la direction de l'axe longitudinal d'élément d'accouplement complémentaire (L2), un mécanisme de déverrouillage (55) par l'intermédiaire duquel la liaison de la structure de fixation côté élément d'accouplement complémentaire (54) avec la structure de fixation côté élément d'accouplement (22) peut être libérée.
